# EUROPEAN PATENT APPLICATION

(11) **EP 4 644 871 A1**
(43) Date of publication of application: **05.11.2025**
(21) Application number: 23911888.8
(22) Date of filing: 20.12.2023
(51) Int. Cl.: G01N 21/64

(54) **IMAGE ACQUISITION DEVICE, IMAGE ACQUISITION METHOD, AND IMAGE ACQUISITION PROGRAM**

(30) Priority: 28.12.2022 JP 2022211086
(71) Applicant: Yokogawa Electric Corporation, Tokyo 180-8750 (JP)
(72) Inventor: MIYAUCHI, Yuki, Musashino-shi, Tokyo 180-8750 (JP); TADENUMA, Takashi, Musashino-shi, Tokyo 180-8750 (JP); TAGUCHI, Tomoyuki, Musashino-shi, Tokyo 180-8750 (JP)
(74) Representative: Winter, Brandl - Partnerschaft mbB
(86) International application number: PCT/JP2023/045694
(87) International publication number: WO 2024/143108

(57) **Abstract**

An image acquiring apparatus 200 acquires a fluorescence image in which a DNA microarray 20 is captured by an imaging device 2 under a first imaging condition using a predetermined excitation wavelength and a predetermined light receiving wavelength, acquires a sample-noise correction image in which the DNA microarray 20 is captured by the imaging device under a second imaging condition in which at least one of an excitation wavelength and a light receiving wavelength is different from that of the first imaging condition, and generates a noise-corrected fluorescence image in which sample noise originating from the DNA microarray 20 included in the fluorescence image is removed by using the sample-noise correction image.

## Description

### Field

The present invention relates to an image acquiring apparatus, an image acquiring method, and an image acquiring program.

### Background

As a method of measuring a target that has specific nucleic acid sequence, such as deoxyribonucleic acid (DNA) contained in a sample, the DNA microarray method that utilizes a DNA microarray is widely known. The DNA microarray method is a method of measuring a target by utilizing the property that a fluorescence-labeled target in the sample added to the DNA microarray is captured by a detection probe of the DNA microarray through a hybridization reaction. In the DNA microarray method, a brightness value or light intensity at a DNA spot area is calculated by image analysis of a fluorescence image, and an amount of the target contained in the sample can be thereby measured in addition to determination whether the target is contained in the sample.

### Citation List

### Patent Literature

Patent Literature 1: JP-A-2008-203138

### Summary

### Technical Problem

However, in the conventional technique, it is difficult to obtain fluorescence images with high detection accuracy in the DNA microarray method. For example, in the conventional technique described above, the DNA spot may become undetectable when the light intensity of the DNA spot area in the fluorescence image becomes lower than a noise in the background.

The present invention has been achieved in view of the above problems, and it is an object of the present invention to obtain a fluorescence image with high detection accuracy.

### Solution to Problem

The present invention provides an image acquiring apparatus including an acquiring unit that acquires a fluorescence image in which a measurement target sample is captured by an imaging device under a first imaging condition using a predetermined excitation wavelength and a predetermined light receiving wavelength, and that acquires a first correction image in which the measurement target sample is captured by the imaging device under a second imaging condition in which at least one of the excitation wavelength and the light receiving wavelength is different from those of the first imaging condition, and a generating unit that generates a noise-corrected fluorescence image in which sample noise originating from the measurement target sample included in the fluorescence image is removed, by using the first correction image.

Moreover, the present invention provides an image acquiring method of a computer performing processing of acquiring a fluorescence image in which a measurement target sample is captured by an imaging device under a first imaging condition using a predetermined excitation wavelength and a predetermined light receiving wavelength,
acquiring a first correction image in which the measurement target sample is captured by the imaging device under a second imaging condition in which at least one of the excitation wavelength and the light receiving wavelength is different from those of the first imaging condition, and generating a noise-corrected fluorescence image in which sample noise originating from the measurement target sample included in the fluorescence image is removed, by using the first correction image.

Moreover, the present invention provides an image acquiring program that causes a computer to execute acquiring a fluorescence image in which a measurement target sample is captured by an imaging device under a first imaging condition using a predetermined excitation wavelength and a predetermined light receiving wavelength,
acquiring a first correction image in which the measurement target sample is captured by the imaging device under a second imaging condition in which at least one of the excitation wavelength and the light receiving wavelength is different from those of the first imaging condition, and generating a noise-corrected fluorescence image in which sample noise originating from the measurement target sample included in the fluorescence image is removed, by using the first correction image. Advantageous Effects of Invention

According to the present invention, an effect is produced that a fluorescence image with high detection accuracy can be obtained.

### Brief Description of Drawings

FIG. 1 is a flowchart illustrating an example of a flow of processing in a DNA microarray method.
FIG. 2 is a diagram for explaining an image acquiring system according to a reference technique.
FIG. 3 is a flowchart for explaining a flow of image acquisition processing according to the reference technique.
FIG. 4 is a diagram for explaining a DNA-microarray fluorescence image according to the reference technique.
FIG. 5 is a diagram for explaining a line profile of a fluorescent spot in a DNA-microarray fluorescence image according to the reference technique.
FIG. 6 is a diagram illustrating a configuration example of an image acquiring system according to an embodiment.
FIG. 7 is a block diagram illustrating a configuration example of an image acquiring apparatus and an imaging device according to the embodiment.
FIG. 8 is a diagram illustrating an example of a three-dimensional fluorescence spectrum used for selecting a device wavelength according to the embodiment.
FIG. 9 is a diagram illustrating an example of a noise-correction-signal-image generation processing according to the embodiment.
FIG. 10 is a diagram illustrating a first specific example of a line profile on a DNA microarray according to the embodiment.
FIG. 11 is a diagram illustrating a second specific example of a line profile on the DNA microarray according to the embodiment.
FIG. 12 is a diagram illustrating an example of a noise reduction effect by sample noise correction and device noise correction according to the embodiment.
FIG. 13 is a flowchart illustrating an example of a flow of image acquisition processing according to the embodiment.
FIG. 14 is a diagram illustrating a hardware configuration according to the embodiment.

### Description of Embodiments

Hereinafter, an image acquiring apparatus, an image acquiring method, and an image acquiring program according to one embodiment of the present invention will be explained in detail with reference to the drawings. The present invention is not limited to embodiments described below.

### [Embodiment]

In the following, a configuration of the image acquiring system, a configuration of the image acquiring apparatus, and flows of respective processing will be sequentially explained, and then an effect of the embodiment will be explained finally.

### [1. Configuration of Image Acquiring System 2000]

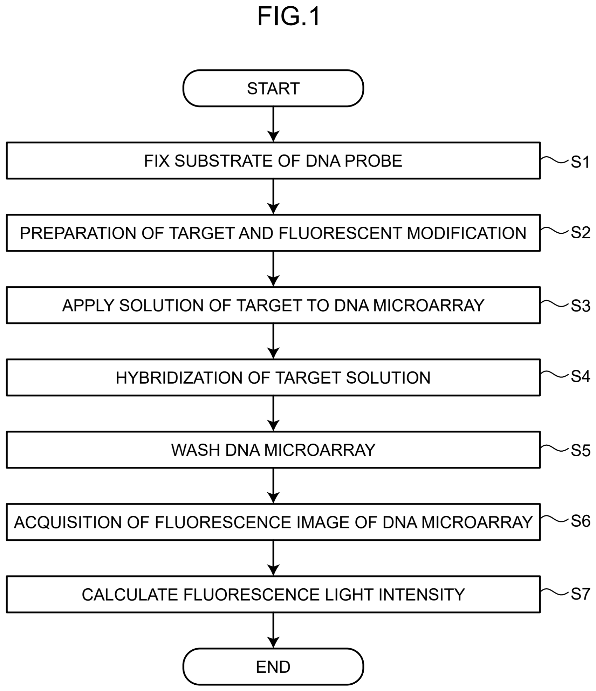

After explaining a flow of processing of a DNA microarray method and an image acquiring system 1000 according to a reference technique, an image acquiring system 2000 according to the embodiment will then be explained in detail using FIG. 1 to FIG. 7. Although an example relating to detection processing of a biomacromolecule measurement device for measuring a specific target molecule out of biomacromolecules contained in a sample by the DNA microarray method will be explained as the image acquiring system 2000 according to the embodiment, a scope of application of the image acquiring system 2000 is not particularly limited.

### (1-1. Flow of Processing of DNA Microarray Method)

A flow of processing of the DNA microarray method underlying the image acquiring system 1000 according to the reference technique and the image acquiring system 2000 according to the embodiment will be explained using FIG. 1. FIG. 1 is a flowchart illustrating an example of the flow of the DNA microarray method. As illustrated in FIG. 1, in the DNA microarray method, processing at steps S1 to S7 in the following are performed.

First, a DNA probe having a complementary sequence to a specific nucleic acid sequence to be a target is immobilized on a solid-phase surface of the substrate of the DNA microarray (step S1). Second, the target in a sample is labeled with a fluorescent molecule, and a target solution is prepared (step S2). Third, the DNA microarray on which the DNA probe is fixed supplies (applies) the prepared target solution to a container arranged inside a container to bring the target solution into contact with the solid-phase surface of the DNA microarray (step S3). Fourth, after bringing the target solution into contact with the solid-phase surface of the DNA microarray on which the DNA probe is fixed, the target labeled with the fluorescent molecule and the DNA probe fixed to the DNA microarray are hybridized (step S4). At this time, by the hybridization reaction at step S4, the target binds with the DNA probe, and the fluorescent molecule attached to the target is captured at the DNA spot at which the DNA probe is fixed. Fifth, by washing the DNA microarray, the target solution is removed, and a target not hybridized and a fluorescent molecule that is not captured are removed (step S5). Sixth, a fluorescence image of the DNA microarray, the fluorescent molecule is captured in the DNA spot is acquired (step S6). Seventh, by calculating a brightness value or a light intensity of the DNA spot area by image analysis of the fluorescence image, whether the target is contained in the sample is determined, or an amount of the target contained in the sample is calculated (step S7).

### (1-2. Image Acquiring System 1000 of Reference Technique)

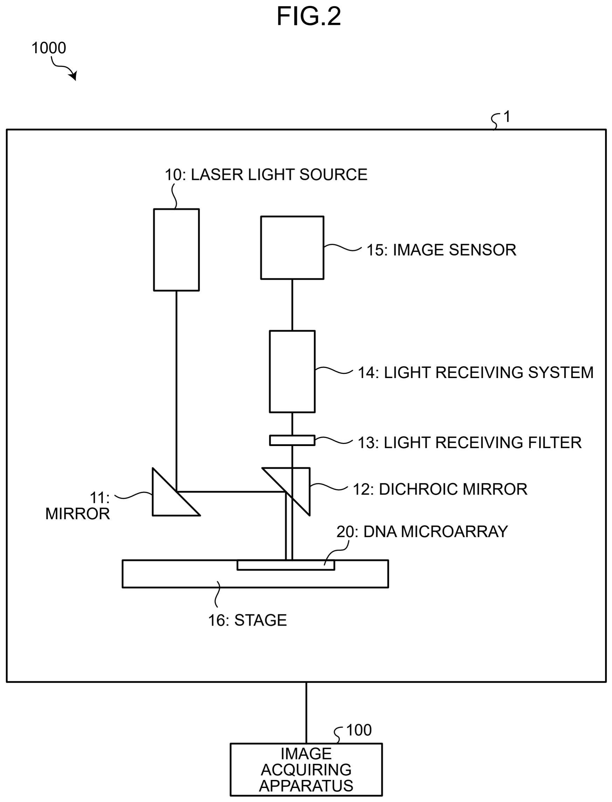

The image acquiring system 1000 according to the reference technique described in Patent Literature 1 will be explained using FIG. 2 to FIG. 5. In the following, a configuration example of an imaging device 1 of the image acquiring system 1000 in the reference technique, a processing example of the image acquiring system 1000, and a problem in the image acquiring system 1000 will be sequentially explained.

### (1-2-1. Configuration Example of Image Acquiring System 1000)

A configuration example of the image acquiring system 1000 according to the reference technique will be explained using FIG. 2. FIG. 2 is a diagram illustrating a configuration example of the image acquiring system 1000 according to the reference technique. The image acquiring system 1000 includes the imaging device 1 and an image acquiring apparatus 100.

The imaging device 1 includes a laser light source 10, a mirror 11, a dichroic mirror 12, a light receiving filter 13, a light-receiving optical system 14, an image sensor 15, and a stage 16. Moreover, the imaging device 1 images a DNA microarray 20 placed on a stage 16 as a measurement target sample.

### (1-2-2. Processing Example of Image Acquiring System 1000)

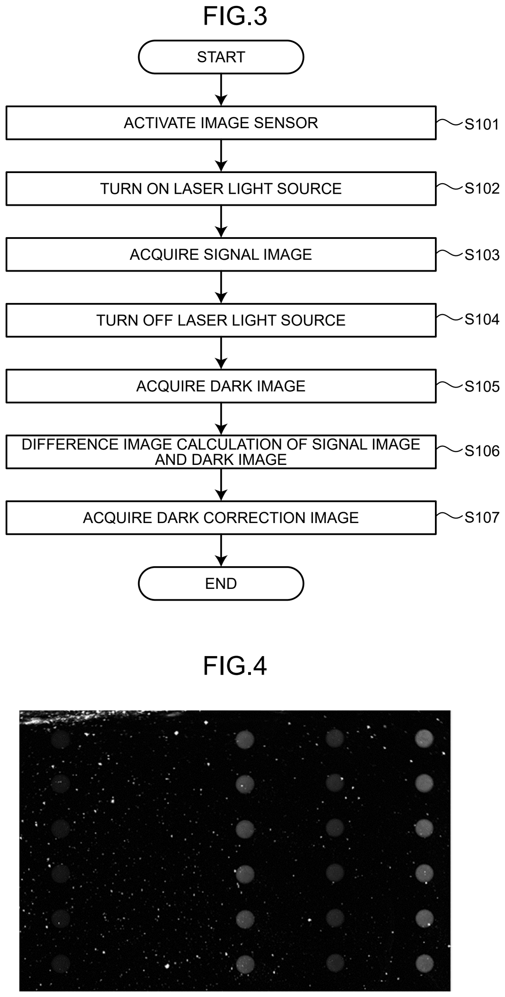

A processing example of the image acquiring system 1000 according to the reference technique will be explained using FIG. 3 to FIG. 5. In the following, fluorescence image acquisition processing and image analysis processing of the image acquiring system 1000 according to the reference technique will be sequentially explained.

### (1-2-2-1. Fluorescence image Acquisition Processing)

Fluorescence image acquisition processing of the image acquiring system 1000 according to the reference technique will be explained using FIG. 3. FIG. 3 is a flowchart for explaining a flow of the image acquisition processing according to the reference technique. The image acquiring system 1000 according to the reference technique performs dark correction by acquiring a dark image through excitation-light ratio irradiation as noise correction. Because a signal image includes a dark noise due to dark current of the image sensor 15, for fluorescence images with low light intensity, it is common to perform dark-noise subtraction processing. As illustrated in FIG. 3, in the fluorescence image acquisition processing of the image acquiring system 1000 according to the reference technique, steps S101 to S107 below are performed.

First, to acquire a fluorescence image of the DNA microarray 20, the imaging device 1 activates the imaging sensor of the imaging device 1 (step S101). Second, the imaging device 1 turns on the laser light source 10 (step S102). At this time, emitted irradiation light is reflected on the mirror 11 and the dichroic mirror 12, to be irradiated to the DNA microarray 20, and is captured by the DNA microarray 20, thereby exciting fluorescent molecules, to radiate fluorescence. Moreover, the fluorescence radiated from the DNA microarray 20 passes through the dichroic mirror 12, and reflection of the irradiation light is removed by the light receiving filter 13, and only the fluorescence is thereby passed through. The passed fluorescence forms an image of the DNA microarray 20 by the light-receiving optical system 14 on a light receiving surface of the image sensor 15. Third, the image acquiring apparatus 100 acquires a fluorescence image as a signal image, by acquiring the image captured by the image sensor 15 (step S103). Fourth, the imaging device 1 turns off the laser light source (step S104). Fifth, the image acquiring apparatus 100 acquires a dark image with the same settings as the signal image (step S105). Sixth, the image acquiring apparatus 100 performs image processing to subtract the dark image from the signal image (step S106). Seventh, the image acquiring apparatus 100 acquires a dark correction image as a fluorescence image subjected to correction (step S107).

### (1-2-2-2. Image Analysis Processing)

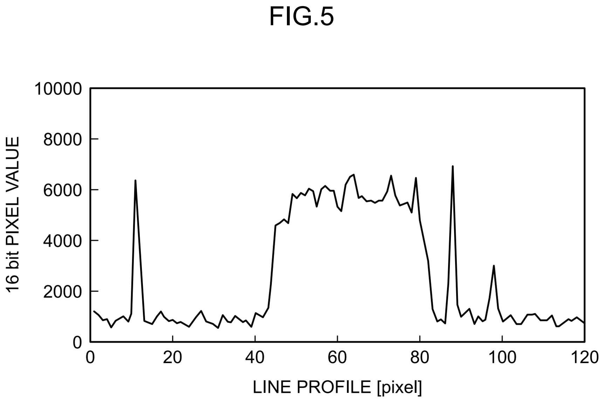

Image analysis processing of the image acquiring system 1000 according to the reference technique will be explained using FIG. 4 and FIG. 5. FIG. 4 is a diagram for explaining the DNA-microarray fluorescence image according to the reference technique. FIG. 5 is a diagram for explaining a line profile of a fluorescent spot in the DNA-microarray fluorescence image according to the reference technique.

In the image analysis processing of the image acquiring system 1000 according to the reference technique, the image acquiring apparatus 100 calculates a brightness value or light intensity of the DNA spot area by image analysis of the acquired dark correction image. At this time, the image acquiring apparatus 100 determines whether a target of the DNA spot as illustrated in FIG. 4 is contained in a sample, or measures an amount of the target contained in the sample. Moreover, for the detection of the DNA spot, the image acquiring apparatus 100 determines based on the brightness value or the light intensity of the DNA spot area being significantly higher than that of the background from a line profile of the image as illustrated in FIG. 5. The image acquiring apparatus 100 can identify an area of the DNA spot by image processing, such as contour extraction and feature extraction.

### (1-2-3. Problems with Image Acquiring System 1000)

In the following, after explaining types of noise to be a cause of decrease in detection accuracy of a fluorescence image, problems with the image acquiring system 1000 will be explained.

### (1-2-3-1. Types of Noise)

In detection of a fluorescence image, when light intensity of a DNA spot area in a fluorescence image becomes lower than noise in the background, the DNA spot can become undetectable. As a factor of noise in the background, there are an absolute value of light intensity for background light and various types of noise. Furthermore, the noise in the background includes sensor noise originating from the image sensor 15, device noise originating from the imaging device 1, and sample noise originating from the DNA microarray 20, which is the sample.

### (Sensor Noise)

Sensor noise includes white noise that randomly exists in an image, and fixed pattern noise that exists in a fixed pattern. The white noise is further divided into a component that varies depending on signal intensity and a component that is not dependent on the signal intensity. The white noise that varies depending on signal intensity includes shot noise due to a signal current. On the other hand, the white noise not dependent on signal intensity includes readout noise and shot noise due to a dark current. Moreover, the fixed pattern noise includes dark current nonuniformity.

### (Device Noise)

The device noise includes fixed pattern noises of unevenness of irradiated light, scattered light from outside the device, stray light inside the device, and autofluorescence from an optical device as the fixed pattern noise.

### (Sample Noise)

The sample noise includes variations in reflected light due to a surface shape of a substrate and fluorescence variations due to autofluorescence of a substrate as the fixed pattern noise.

### (1-2-3-2. Problems)

The image acquiring system 1000 according to the reference technique has following problems. First, in the image acquiring system 1000, while the dark current nonuniformity, which is fixed pattern noise of the sensor noise, is corrected, other fixed pattern noise is so large that imposes a limitation on a lower detection limit. Second, in the image acquiring system 1000, because the white noise occurs randomly over time, convolution of noise can be performed by extending the exposure time and by accumulating images, but for a fluorescence image with low light intensity and the like, exposure time becomes longer with existing image sensors such as cooled CCDs (charge coupled devices).

### (1-3. Image Acquiring System 2000)

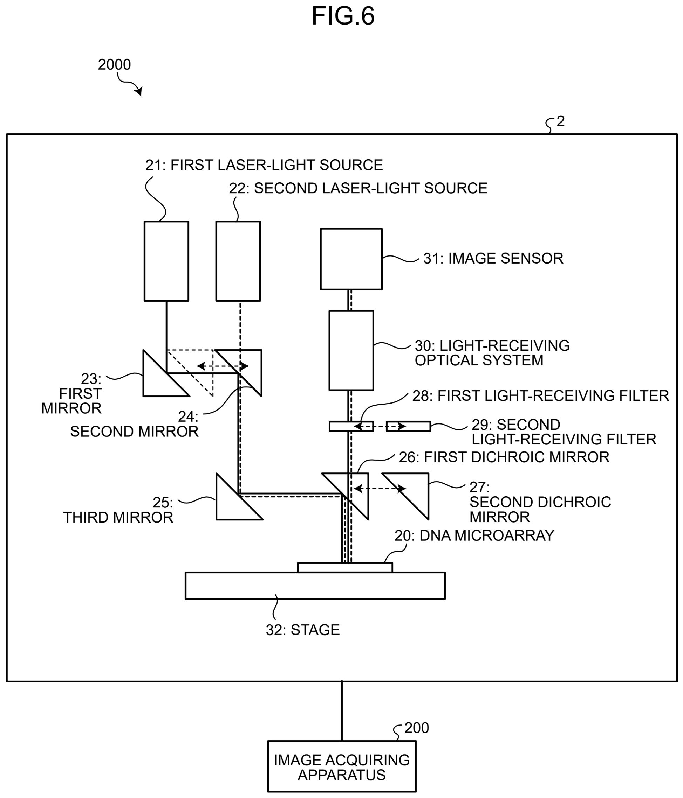

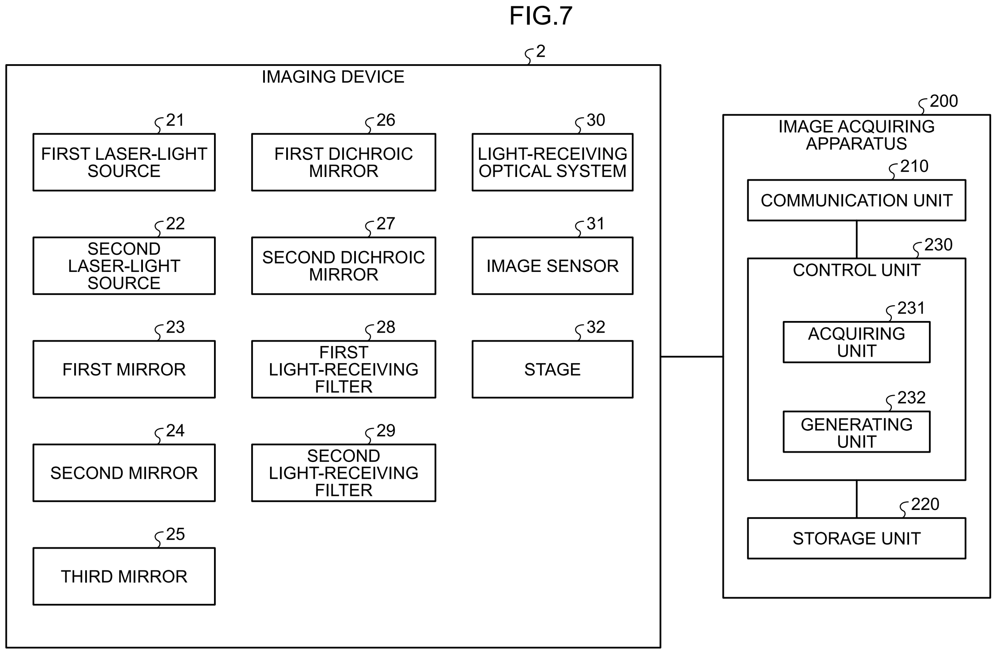

The image acquiring system 2000 according to the embodiment will be explained using FIG. 6 and FIG. 7. In the following, a configuration example of the image acquiring system 2000, a processing example of the image acquiring system 2000, and an effect of the image acquiring system 2000 will be explained sequentially.

### (1-3-1. Configuration Example of Image Acquiring System 2000)

A configuration example of the image acquiring system 2000 according to the embodiment will be explained using FIG. 6. FIG. 6 is a diagram illustrating a configuration example of the image acquiring system 2000 according to the embodiment. The image acquiring system 2000 includes an imaging device 2 and an image acquiring apparatus 200. The image acquiring system 2000 illustrated in FIG. 6 may include multiple units of the imaging devices 2 or multiple units of the image acquiring apparatuses 200. Moreover, the image acquiring apparatus 200 may be configured to be integrated with the imaging device 2.

The imaging device 2 includes a first laser-light source 21, a second laser-light source 22, a first mirror 23, a second mirror 24, a third mirror 25, a first dichroic mirror 26, a second dichroic mirror 27, a first light-receiving filter 28, a second light-receiving filter 29, a light-receiving optical system 30, an image sensor 31, and a stage 32. Moreover, the imaging device 2 images the DNA microarray 20 that is a measurement target sample (referred to as "measurement target sample" as appropriate) placed on the stage 32, or a sample for device noise correction (referred to as "correction sample" as appropriate) not illustrated, placed on the stage 32.

### (1-3-2. Processing Example of Image Acquiring System 2000)

A processing example of the image acquiring system 2000 according to the embodiment will be explained using FIG. 6. In the following, image-data acquisition processing, noise removal processing, and image analysis processing of the image acquiring system 2000 according to the embodiment will be explained sequentially.

### (1-3-2-1. Image-Data Acquisition Processing)

In the image acquiring system 2000 according to the embodiment, the image acquiring apparatus 200 performs image-data acquisition processing. In the following, the image-data acquisition processing of a signal image I1 that is a fluorescence image before noise removal, a device-noise correction image for a signal image (referred to as "second correction image" as appropriate) I2, a sample-noise correction image (referred to as "first correction image" as appropriate) I3, a device-noise correction image for a sample-noise correction image (referred to as "third correction image" as appropriate) I4, a dark image (referred to as "fourth correction image" as appropriate) I5 will be explained.

### (Dark-Image Acquisition Processing)

First, the image acquiring system 2000 performs dark-image acquisition processing as the image-data acquisition processing. First, the imaging device 2 activates the image sensor 31. Next, the imaging device 2 places a sample for device-noise correction on the stage 32. Moreover, the imaging device 2 turns off the first laser-light source (referred to as "first light source" as appropriate) 21, which is a laser light source for the signal image, and the second laser-light source (referred to as "second light source" as appropriate) 22, which is a laser light source for the sample-noise correction image. The image acquiring apparatus 200 acquires image data of the dark image (fourth correction image) I5 that is captured under an imaging condition (third imaging condition) in which the first laser-light source 21 and the second laser-light source 22 are off described above. That is, the image data of the dark image is image data acquired by capturing a device- noise correction sample or the DNA microarray 20 under a condition in which excitation light is not irradiated from the first laser-light source 21 and the second laser-light source 22.

### (Acquisition Processing of Device-Noise Correction Image for Signal Image)

Second, the image acquiring system 2000 performs the acquisition processing of a device-noise correction image for a signal image as the image data acquisition processing. First, the imaging device 2 turns on the first laser-light source 21, and turns off the second laser-light source 22. At this time, the imaging device 2 arranges the second mirror 24 at a solid line position illustrated in FIG. 6 such that emitted light from the first laser-light source 21 irradiates the stage 32. Moreover, the imaging device 2 arranges the first dichroic mirror 26, which is a dichroic mirror for a signal image, as the dichroic mirror, and the first light-receiving filter (referred to as "first filter" as appropriate) 28, which is a light receiving filter for a signal image, as the light receiving filter. The image acquiring apparatus 200 acquires the device-noise correction image (second correction image) I2 for a signal image that is acquired by capturing a sample for device noise correction under an imaging condition (first imaging condition) in which the first laser-light source 21 is on described above. That is, the device-noise correction image for a signal image I2 is image data that is acquired by capturing the device-noise correction sample under the first condition in which excitation light from the first laser-light source 21 is irradiated. In the first condition, a path of the excitation light and fluorescence is indicated by a solid line in FIG. 6.

### (Acquisition Processing of Device-Noise Correction Image for Sample-Noise Correction Image)

Third, the image acquiring system 2000 performs the acquisition processing of a device-noise correction image for a sample-noise correction image as the image data acquisition processing. First, the imaging device 2 turns off the first laser-light source 21, and turns on the second laser-light source 22. At this time, the imaging device 2 arranges the second mirror 24 at a broken line position illustrated in FIG. 6 such that emitted light from the second laser-light source 22 irradiates the stage 32. Moreover, the imaging device 2 arranges the second dichroic mirror 27, which is a dichroic mirror for a sample-noise correction image, as the dichroic mirror, and the second light-receiving filter (referred to as "second filter" as appropriate) 29, which is a light receiving filter for a sample-noise correction image, as the light receiving filter. The image acquiring apparatus 200 acquires the device-noise correction image (third correction image) I4 for a sample-noise correction image that is acquired by capturing a sample for device noise correction under an imaging condition (second imaging condition) in which the second laser-light source 22 is on described above. That is, the device-noise correction image for a sample-noise correction image I4 is image data that is acquired by capturing the device-noise correction sample under the second condition in which excitation light from the second laser-light source 22 is irradiated. In the second condition, a path of the excitation light and fluorescence is indicated by a broken line in FIG. 6.

### (Acquisition Processing of Sample-Noise Correction Image)

Fourth, the image acquiring system 2000 performs the acquisition processing of a sample-noise correction image as the image data acquisition processing. First, the imaging device 2 places the DNA microarray 20, which is a measurement target sample, on the stage 32, replacing the device-noise correction sample. Subsequently, the image acquiring apparatus 200 acquires the sample-noise correction image (first correction image) I3 acquired by capturing the DNA microarray 20 under the second imaging condition in which the second laser-light source 22 is on, while maintaining the apparatus configuration with which the device-noise correction image for a sample-noise correction image (third correction image) I4 described above is acquired (the second mirror 24: broken line position, the second dichroic mirror 27 used, and the second light-receiving filter 29 used). That is, the sample-noise correction image I3 is image data that is acquired by capturing the DNA microarray 20 under the second imaging condition in which excitation light is irradiated from the second laser-light source 22. Under the second imaging condition, a path of the excitation light and fluorescence is indicated by a broken line in FIG. 6.

### (Acquisition Processing of Signal Image)

Fifth, the image acquiring system 2000 performs the acquisition processing of a signal image as the image data acquisition processing. First, the imaging device 2 changes the apparatus configuration to that with which the image data of the device-noise correction image for a signal image (second correction image) I2 is acquired (the second mirror 24: solid line position, the first dichroic mirror 26 used, the first light-receiving filter 28 used), keeping the DNA microarray 20, which is a measurement target sample, on the stage 32. The image acquiring apparatus 200 acquires the signal image I1, which is a fluorescence image and in which the DNA microarray 20 is captured under the first imaging condition with the first laser-light source 21 turned on. That is, the signal image I1 is image data acquired by capturing the DNA microarray 20 under the first imaging condition in which the excitation light is irradiated by the first laser-light source 21. In the first condition, a path of the excitation light and fluorescence is indicated by a solid line in FIG. 6.

### (1-3-2-2. Noise Removal Processing)

In the image acquiring system 2000 according to the embodiment, the image acquiring apparatus 200 performs the noise removal processing. That is, the image acquiring apparatus 200 performs arithmetic processing for noise correction from the acquired signal image I1, the device-noise correction image for a signal image I2, the sample-noise correction image I3, the device-noise correction image for a sample-noise correction image I4, and the dark image I5, and generates a noise-corrected signal image (referred to as "noise-corrected fluorescence image" as appropriate) I12 in which noise is removed. Moreover, the image acquiring apparatus 200 can also acquire image data that is captured by the imaging device 2 equipped with a high sensitivity sensor as the image sensor 31, and reduce white noise.

### (1-3-2-3. Image Analysis Processing)

In the image acquiring system 2000, the image acquiring apparatus 200 calculates a brightness value and light intensity of a DNA spot area by image analysis of the generated noise-corrected signal image 112. At this time, the image acquiring apparatus 200 determines whether a target in the DNA spot is contained in the sample, and measures an amount of the target contained in the sample. Moreover, for the detection of the DNA spot, the image acquiring apparatus 200 determines based on the brightness value or the light intensity of the DNA spot area being significantly higher than that of the background from a line profile of the noise-corrected signal image I12. The image acquiring apparatus 200 can identify an area of the DNA spot by image processing, such as contour extraction and feature extraction, instead of the detection method of a DNA spot using a line profile described above.

### (1-4. Effect of Image Acquiring System 2000)

In the following, after explaining an overview of the image acquiring system 2000 according to the embodiment, an effect of the image acquiring system 2000 will be explained.

### (1-4-1. Overview)

In the image acquiring system 2000, the image acquiring apparatus 200 acquires image data of the signal image I1, which is a fluorescence image captured by the imaging device 2 before noise removal, the device-noise correction image for a signal image I2, the sample-noise correction image I3, the device-noise correction image for a sample-noise correction image I4, and the dark image I5, and generates the noise-corrected signal image 112, which is a fluorescence image in which sample noise and device noise are removed using the images I1 to I5 described above. Moreover, the image acquiring apparatus 200 can acquire image data captured by the imaging device 2 equipped with a high sensitivity sensor, and reduce white noise.

### (1-4-2. Effect)

First, in the image acquiring system 2000, sample noise originating from the DNA microarray 20, which is the sample, can be removed. That is, the image acquiring apparatus 200 acquires a reference image for sample noise correction to remove effects of fixed pattern noise in sample noise, such as variations in reflected light due to a substrate surface shape and variations in fluorescence due to autofluorescence of the substrate, and remove the sample noise by calculation using the acquired sample-noise correction reference image, thereby improving the detection sensitivity.

Second, in the image acquiring system 2000, device noise originating from the imaging device 2 can be removed. That is, the image acquiring apparatus 200 acquires a reference image for device noise correction to remove effects of fixed pattern noise in device noise, such as unevenness of the irradiated light, scattered light from outside the device, stray light inside the device, and autofluorescence from an optical device, and removes the device noise by calculation using the acquired device-noise correction reference image, thereby improving the detection sensitivity.

Third, in the image acquiring system 2000, in addition to the sample noise and the device noise described above, the dark noise can be removed. That is, to remove effects of dark current nonuniformity, which is fixed pattern noise in sensor noise, the image acquiring apparatus 200 acquires a dark image for dark correction, and reduces the fixed pattern noise in sensor noise by calculation using the acquired dark image, thereby improving the detection sensitivity.

Fourth, in the image acquiring system 2000, in addition to the sample noise, the device noise, and the dark noise described above, white noise can be removed. That is, to reduce effects of readout noise and shot noise, which is white noise not dependent on signal intensity in sensor noise, shot noise due to a dark current, shot noise due to a signal current, which is white noise varying depending on signal intensity in sensor noise, the image acquiring apparatus 200 applies a high sensitivity sensor to the imaging device 2 for a DNA microarray image, thereby reducing imaging time.

As described above, in the image acquiring system 2000, by removing various kinds of noise, it becomes possible to acquire a fluorescence image with high detection accuracy in the DNA microarray method.

### [2. Configuration of Respective Devices of Image Acquiring System 2000]

A functional configuration of respective devices included in the image acquiring system 2000 illustrated in FIG. 6 will be explained using FIG. 7 to FIG. 12. In the following, a configuration example of the image acquiring apparatus 200 according to the embodiment, a configuration example of the imaging device 2, and a specific example of respective processing will be sequentially explained in detail.

### (2-1. Configuration Example of Image Acquiring Apparatus 200)

First, a configuration example of the image acquiring apparatus 200 illustrated in FIG. 6 will be explained using FIG. 7. FIG. 7 is a block diagram illustrating a configuration example of the image acquiring apparatus 200 and the imaging device 2 according to the embodiment. The image acquiring apparatus 200 includes a communication unit 210, a storage unit 220, and a control unit 230. The image acquiring apparatus 200 may include an input unit (for example, a keyboard, a mouse, and the like) that accepts various operations from an administrator and the like of the image acquiring apparatus 200, and a display unit (for example, a liquid crystal display, and the like) to display various kinds of information.

### (2-1-1. Communication Unit 210

The communication unit 210 is responsible for data communication with other devices. For example, the communication unit 210 performs data communication with respective communication devices through a router and the like. Moreover, the communication unit 210 can perform data communication with an operator terminal not illustrated.

### (2-1-2. Storage Unit 220)

The storage unit 220 stores various kinds of information referred to when the control unit 230 operates, and various kinds of information acquired when the control unit 230 operates. The storage unit 220 can be implemented by a semiconductor memory device, such as a RAM (random access memory) and a flash memory, or a storage device such as a hard disk and an optical disk. In the example in FIG. 7, the storage unit 220 is arranged inside the image acquiring apparatus 200, but it may be arranged outside the image acquiring apparatus 200, and more than one storage unit may be arranged.

The storage unit 220 stores image data acquired by an acquiring unit 231 of the control unit 230 described later. For example, the storage unit 220 stores image data of the signal image I1, which is a fluorescence image before noise removal, acquired from the imaging device 2, the device-noise correction image 12 for a signal image, the sample-noise correction image I3, the device-noise correction image for a sample-noise correction image, the dark image I5, and the like.

Moreover, the storage unit 220 stores image data that is generated by a generating unit 232 of the control unit 230 described later. For example, the storage unit 220 stores a dark-corrected signal image 16, a dark-corrected device-noise correction image for a signal image I7, a dark-corrected sample-noise correction image 18, a dark-corrected device-noise correction image for a sample-noise correction image I9, a device-noise-corrected signal image 110, a device-noise-corrected sample-noise correction image I11, the noise-corrected signal image 112, and the like.

### (2-1-5. Control Unit 230)

The control unit 230 is responsible for overall control of the image acquiring apparatus 200. The control unit 230 includes the acquiring unit 231 and the generating unit 232. The control unit 230 can be implemented by an electronic circuit, such as a CPU (central processing unit) and a MPU (micro processing unit), or an integrated circuit, such as an ASIC (application specific integrated circuit) and a FPGA (field programmable gate array).

### (2-1-5-1. Acquiring Unit 231)

The acquiring unit 231 acquires various kinds of images captured by the imaging device 2. The acquiring unit 231 may store the acquired various images in the storage unit 220. In the following, acquisition processing of the signal image I1, which is a fluorescence image, acquisition processing of the sample-noise correction image I3, which is the first correction image, acquisition processing of the device-noise correction image for a signal image I2, which is the second correction image, acquisition processing of the device-noise correction image for a sample-noise correction image I4, which is the third correction image, acquisition processing of the dark image I5, which is the fourth correction image, and acquisition processing of the white-noise correction image will be explained sequentially.

### (Acquisition Processing of Signal Image I1)

The acquiring unit 231 acquires the signal image I1, which is a fluorescence image in which a measurement target sample is captured by the imaging device 2 under the first imaging condition in which use of a predetermined excitation wavelength and a predetermined light reception wavelength is selected. For example, the acquiring unit 231 acquires the signal image I1 in which the DNA microarray 20 being the measurement target sample is captured by the imaging device 2 under the first imaging condition in which the first laser-light source 21 irradiating excitation light of a predetermined wavelength and the first light-receiving filter 28 being the first filter that selects fluorescence of a predetermined wavelength are used.

To explain with a specific example, the acquiring unit 231 acquires the signal image I1 in which the DNA microarray 20 is captured by the imaging device 2 under an imaging condition in which the first laser-light source 21 irradiating excitation light with an excitation wavelength of 532 nm and the first light-receiving filter 28 that selects fluorescence with a light reception wavelength of 600±37.5 nm are arranged.

### (Acquisition Processing of Sample-Noise Correction Image 13)

The acquiring unit 231 acquires the sample-noise correction image I3, which is the first correction image, capturing a measurement target sample by the imaging device 2 under the second imaging condition in which at least one of the excitation wavelength and the light reception wavelength is different from the first imaging condition. For example, the acquiring unit 231 acquires the sample-noise correction image I3 in which the DNA microarray 20 being the measurement target sample is captured by the imaging device 2 under the second imaging condition in which at least one of the second laser-light source 22 being the second light source irradiating excitation light of a wavelength different from that of the first laser-light source 21 being the first light source, and the second light-receiving filter 29 being the second filter selecting fluorescence having a wavelength different from that of the first light-receiving filter 28 being the first filter is used.

To explain with a specific example, the acquiring unit 231 acquires the sample-noise correction image I3 in which the DNA microarray 20 is captured by the imaging device 2 under the second imaging condition in which the excitation wavelength is 532 nm, which is identical to the first imaging condition, and the light reception wavelength is 700±37.5 nm, which is different from the first imaging condition.

### (Acquisition Processing of Device-Noise Correction Image for Signal Image I2)

The acquiring unit 231 acquires the device-noise correction image for a signal image I2, which is the second correction image capturing a correction sample different from the measurement target sample by the imaging device 2 under the first imaging condition. For example, the acquiring unit 231 acquires a device-noise correction image for a sample-noise correction image I4I2 in which a sample not containing fluorescence emitted by the DNA microarray 20 being the measurement target sample or a mirror is captured by the imaging device 2 under the first imaging condition.

To explain with a specific example, the acquiring unit 231 acquires the device-noise correction image I2 for a signal image in which a high surface precision mirror being a sample for correction is captured by the imaging device 2 under the first imaging condition in which the excitation wavelength of 532 nm and the light receiving wavelength of 600±37.5 nm are selected.

### (Acquisition Processing of Device-Noise Correction Image for Sample-Noise Correction Image I4)

The acquiring unit 231 acquires the device-noise correction image for a sample-noise correction image I4, which is the third correction image in which a correction sample is captured by the imaging device 2 under the second imaging condition. For example, the acquiring unit 231 acquires the device-noise correction image for a sample-noise correction image I4 in which a sample not containing fluorescence emitted by the DNA microarray 20 being the measurement target sample, or a mirror is captured as the sample for correction by the imaging device 2 under the second imaging condition.

To explain with a specific example, the device-noise correction image for a sample-noise correction image I4 is acquired in which a high surface precision mirror being a sample for correction is captured by the imaging device 2 under the second imaging condition in which the excitation wavelength is 532 nm identical to that of the first imaging condition and the light-receiving wavelength is 700±37.5 nm different from that of the first imaging condition.

### (Acquisition Processing of Dark Image I5)

The acquiring unit 231 acquires the fourth correction image captured by the imaging device 2 under the third imaging condition in which excitation light is not irradiated. For example, the acquiring unit 231 acquires the fourth correction image in which a measurement target sample or a sample for correction is captured by the imaging device 2 under the third imaging condition in which excitation light is not irradiated neither from the first laser-light source 21, which is the first light source nor the second laser-light source 22, which is the second light source.

To explain with a specific example, the acquiring unit 231 acquires the dark image I5 in which a high-precision surface mirror, which is the sample for correction, is captured by the imaging device 2 under the third imaging condition in which the first laser-light source 21 and the second laser-light source 22 are both turned off so that the excitation light is not irradiated.

### (Acquisition Processing of White-Noise Removal Image)

The acquiring unit 231 acquires the signal image I1, which is a fluorescence image captured using the imaging device 2 having the image sensor 31 that applies gain to the signal so that intensity of light emitted by the DNA microarray 20, which is the measurement target sample, is equal to or higher than readout noise, or the image sensor 31 in which readout noise is equal to or lower than a predetermined value.

### (2-1-5-2. Generating Unit 232)

The generating unit 232 generates a noise-removed image from which various kinds of noise have been removed from various kinds of images acquired by the acquiring unit 231. The generating unit 232 may store the generated noise-removed image in the storage unit 220. In the following, after respective noise removal processing is explained in order of sample-noise removal processing for the signal image I1, device-noise removal processing for the signal image I1, device-noise removal processing for the device-noise correction image for a signal image I2, and dark-noise removal processing, overall noise removal processing will be explained.

### (Sample-Noise Removal Processing of Signal Image I1)

The generating unit 232 removes sample noise originating from a measurement target sample contained in the signal image I1, which is fluorescence image, by using the sample-noise correction image I3, which is the first correction image.

### (Device-Noise Removal Processing for Signal Image I1)

The generating unit 232 removes device noise originating from the imaging device 2 contained in the signal image I1, which is a fluorescence image, by using the device-noise correction image for a signal image 12, which is the second correction image.

### (Device-Noise Removal Processing for Device-Noise Correction Image I2 for Signal Image)

The generating unit removes device noise contained in the sample-noise correction image I3, which is the first correction image, by using the device-noise correction image for a sample-noise correction image I4, which is the third correction image.

### (Dark-Noise Removal Processing)

The generating unit 232 removes the dark noise originating from dark current of the imaging device 2 contained in the signal image I1, which is a fluorescence image, the sample-noise correction I3, which is the first correction image, the device-noise correction image for a signal image I2, which is the second correction image, and the device-noise correction image for a sample-noise correction image I4, which is the third correction image.

### (Overall Noise-Removal Processing)

First, the generating unit 232 generates, by performing dark-image subtraction processing using the dark image I5, the dark-correction signal image I6 from the signal image I1, the dark-corrected sample-noise correction image I8 from the sample-noise correction image I3, the dark-corrected device-noise correction image for a signal image I7 from the device-noise correction image for a signal image I2, and the dark-corrected device-noise correction image I9 for a sample-noise correction image from the device-noise correction image for a sample-noise correction image I4.

Second, the generating unit 232 calculates a device-noise correction coefficient for a signal image, which is a first device-noise correction coefficient, based on a brightness value or light intensity from the dark-corrected device-noise correction image for a signal image I7, and divides dark-corrected signal image I6 by the device-noise correction coefficient for a signal image, thereby generating the device-noise corrected fluorescence image I10 from which device noise is removed from the signal image I1.

Third, the generating unit 232 calculates a device-noise correction coefficient for a sample-noise correction image, which is a second device-noise correction coefficient, based on a brightness value or light intensity from the dark-corrected device-noise correction image for a sample-noise correction image I9, and divides dark-corrected sample-noise correction image I8 by the second device-noise correction coefficient, thereby generating the device-noise-corrected sample-noise correction image I11, which is the fifth correction image from which device noise is removed from the sample-noise correction image I3.

Fourth, the generating unit 232 divides the device-noise corrected fluorescence image I10 by the device-noise-corrected sample-noise correction image I11, thereby generating the noise-correction fluorescence image I12 that is obtained by removing the sample noise from the device-noise corrected fluorescence image I10.

### (2-2. Configuration Example of Imaging Device 2)

A configuration example of the imaging device 2 illustrated in FIG. 6 will be explained using FIG. 7. The imaging device 2 includes the first laser-light source 21, the second laser-light source 22, the first mirror 23, the second mirror 24, the third mirror 25, the first dichroic mirror 26, the second dichroic mirror 27, the first light-receiving filter 28, the second light-receiving filter 29, the light-receiving optical system 30, the image sensor 31, and the stage 32.

### (2-2-1. First Laser-Light Source 21)

The first laser-light source 21 is a laser light source for a signal image, and irradiates laser light to the DNA microarray 20 or a device-noise correction sample placed on the stage 32 under the first imaging condition. For example, the first laser-light source 21 is implemented by a laser light source that emits single wavelength laser light or expanded light of the laser light, a LED (light emitting diode), a lamp that emits white light, a light source composed of a combination of an LED and a wavelength filter, and the like.

### (2-2-2. Second Laser-Light Source 22)

The second laser-light source 22 is a laser light source for a sample-noise correction image, and irradiates laser light to the DNA microarray 20 or the device-noise correction sample on the stage 32 under the second imaging condition. For example, the second laser-light source 22 can be implemented by a laser light source that emits single wavelength laser light or expanded light of the laser light, an LED, a lamp that emits white light, a light source composed of a combination of an LED and a wavelength filter, and the like.

### (2-2-3. First Mirror 23)

The first mirror 23 guides the laser light emitted from the first laser-light source 21 to the second mirror 24.

### (2-2-4. Second Mirror 24)

The second mirror 24 guides laser light emitted from the first laser-light source 21 and reflected by the first mirror 23 to the third mirror 25.

### (2-2-5. Third Mirror 25)

The third mirror 25 guides laser light that is emitted from the first laser-light source 21, reflected by the first mirror 23, and reflected by the second mirror 24 to the first dichroic mirror 26. Moreover, the third mirror 25 guides laser light emitted from the second laser-light source 22 to the second dichroic mirror 27.

### (2-2-6. First Dichroic Mirror 26)

The first dichroic mirror 26 guides laser light that is emitted from the first laser-light source 21, reflected by the first mirror 23, reflected by the second mirror 24, and reflected by the third mirror 25 to the stage 32.

### (2-2-7. Second Dichroic Mirror 27)

The first dichroic mirror 26 guides laser light that is emitted from the second laser-light source 22, and reflected by the third mirror 25 to the stage 32.

### (2-2-8. First Light-Receiving Filter 28)

The first light-receiving filter 28 guides fluorescence that is excited by laser light emitted from the first laser-light source 21 and radiated from the DNA microarray 20 or a sample for correction to the light-receiving optical system 30. At this time, the first light-receiving filter 28 removes reflection of the laser light, which is irradiated light, and passes only fluorescence.

### (2-2-9. Second Light-Receiving Filter 29)

The second light-receiving filter 29 guides fluorescence that is excited by laser light emitted from the second laser-light source 22 and radiated from the DNA microarray 20 or a device-noise correction sample to the light-receiving optical system 30. At this time, the second light-receiving filter 29 removes reflection of the laser light, which is irradiated light, and passes only fluorescence.

### (2-2-10. Light-Receiving Optical System 30)

The light-receiving optical system 30 guides the fluorescence that has passed through the first light-receiving filter 28 to the image sensor 31. Moreover, the light-receiving optical system 30 guides the fluorescence that has passed through the second light-receiving filter 29 to the image sensor 31.

### (2-2-11. Image Sensor 31)

The image sensor 31 converts fluorescence imaged by the light-receiving optical system 30 into an electrical signal. For example, the image sensor 31 can be implemented by an EM (electron multiplying)-CCD, a CMOS (complementary metal-oxide semiconductor), and the like.

### (2-2-12. Stage 32)

The stage 32 fixes the DNA microarray 20 or a device-noise correction sample.

### (2-3. Specific Example of Various Kinds of Processing)

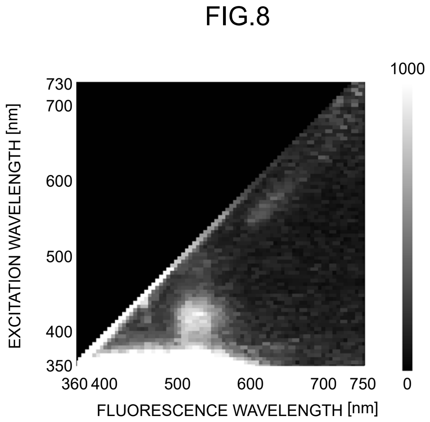

A specific example of various kinds of processing of the image acquiring system 2000 according to the embodiment will be explained using FIG. 8 to FIG. 12. In the following, wavelength selection in the acquisition processing of the signal image I1, wavelength selection in the acquisition processing of the sample-noise correction image I3, the acquisition processing of the device-noise correction image, image processing calculation in the noise removal processing, white-noise removal processing, confirmation of noise reduction effect, the image-data acquisition processing, and a modification of the noise-removal processing will be sequentially explained.

### (2-3-1. Wavelength Selection in Acquisition Processing of Signal Image I1)

In the following, the wavelength selection in the acquisition processing of the signal image I1 will be explained. In the acquisition processing of the signal image I1, for the first laser-light source 21, which is the laser light source for a signal image, for example, a laser light source that emits a single wavelength laser light or expanded light of the laser light, an LED, a lamp that emits white light, a light constituted of a combination of an LED and a wavelength filter, and the like can be used. Generally, when white light is used, a band-pass filter is used to extract only a wavelength range corresponding to an excitation wavelength. However, if light of a wavelength band that is supposed to be cut originally by a filter is contained in excitation light without being cut due to imperfection in non-transmissivity of the filter, and if the wavelength of the transmitted excitation light overlaps with a fluorescence wavelength, it becomes a factor of background light. Therefore, in high sensitivity measurement, a laser light source in which an excitation wavelength band of a light source is narrow is often used. Meanwhile, when using a laser light source, because commercially available wavelengths are limited, the wavelength is to be selected from those available on the market.

In wavelength selection of a light source, a dichroic mirror, and a light receiving filter, it is desirable to select a wavelength that has a high S/N ratio of noise N including shot noise that increases according to a background light intensity B based on the wavelength characteristics of the background light and detection target fluorescent molecules as appropriate and light intensity S of a DNA spot in which fluorescent molecules gather, and that is detectable.

### (2-3-2. Wavelength Selection in Acquisition Processing of Sample-Noise Correction Image I3)

In the following, wavelength selection in the acquisition processing of the sample-noise correction image I3 will be explained. The light source is selected from among various light sources similarly to the acquisition processing of the signal image I1. That is, for the second laser-light source 22, which is the laser light source for a sample-noise correction image, for example, a laser light source that emits a single wavelength laser light or expanded light of the laser light, an LED, a lamp that emits white light, a light constituted of a combination of an LED and a wavelength filter, and the like can be used.

The sample-noise correction image I3 is acquired for the purpose of removing nonuniform reflection caused by a surface shape of a substrate and nonuniform fluorescence in autofluorescence of the substrate that are present as the fixed pattern noise. A wavelength is preferable at which fluorescence of fluorescence molecules to be a signal is not detected, and at which nonuniform reflection caused by the surface shape of the substrate and nonuniform fluorescence in autofluorescence of the substrate are detected. Generally, a fluorescence wavelength of a fluorescent molecule has a spectrum with a distinct peak, and materials, such as glass and resin, used for the substrate are considered to have a broad fluorescence spectrum. By obtaining an excitation light source with a wavelength that avoids the excitation wavelength band of the fluorescent molecule and a detection filter that avoids the fluorescence wavelength band, an aimed image can be acquired.

A three-dimensional fluorescence spectrum used for selection of a device wavelength will be explained using FIG. 8. FIG. 8 is a diagram illustrating an example of the three-dimensional fluorescence spectrum used for selection of a device wavelength according to the embodiment. In the example in FIG. 8, a three-dimensional fluorescence spectrum of a glass substrate is illustrated. To acquire an image in which nonuniform reflection caused by a surface shape of the substrate and nonuniform fluorescence in autofluorescence of the substrate are reflected, in a range outside the fluorescence wavelength band of a fluorescent molecule in the three-dimensional fluorescence spectrum, an excitation wavelength and a light receiving wavelength can also be selected. Moreover, because it can be the range outside the fluorescence wavelength band of a fluorescence molecule, it is not necessary to change both the excitation wavelength and the light receiving wavelength from those in the signal image acquisition, and only by changing either one, an aimed image can be acquired.

### (2-3-3. Acquisition Processing of Device-Noise Correction Image)

In the following, the acquisition processing of a device-noise correction image common to the acquisition processing of the device-noise correction image for a signal image I2 and the acquisition processing of the device-noise correction image for a sample-noise correction image I4 will be explained. The device-noise correction images I2, I4 are acquired for the purpose of removing effects of unevenness of the irradiated light, scattered light from outside the device, stray light inside the device, and autofluorescence from an optical device that exist as the fixed pattern noise. The device-noise correction image is preferable to be an image that reflects effects of the device alone, not detecting fluorescence of a fluorescence molecule to be a signal, and not detecting nonuniform reflection caused of a surface shape of the substrate and nonuniform fluorescence in autofluorescence of the substrate.

As the device-noise correction samples I2, I4, which are the correction sample, not to reflect the surface shape of the substrate, an average image obtained by capturing multiple points in the DNA microarray 20 in which a fluorescence molecule is not captured, a high surface-precision mirror, or the like can be used. Because optical devices are changed between an optical system for a signal image acquisition and an optical system for a sample-noise correction image acquisition, it is preferable to acquire for each. Moreover, there are many of long-period fixed pattern noise, such as unevenness of the irradiated light included in the device-noise correction images I2, I4, and in this case, it is smoothing and application of an image filter is also effective.

### (2-3-4. Image Processing Calculation in Noise Removal Processing)

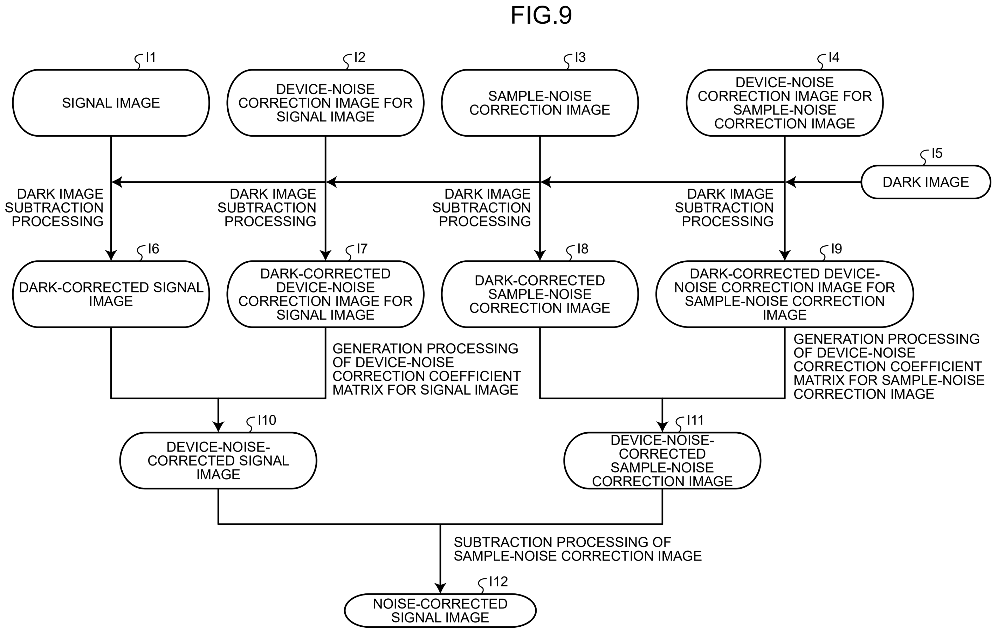

The image processing calculation in the noise removal processing will be explained using FIG. 9. It is a diagram illustrating an example of the noise-correction-signal-image generation processing according to the embodiment. In the example in FIG. 9, a flow of the image processing calculation to generate the noise-correction image I12 from the acquired signal image I1, the sample-noise correction image I3, the device-noise correction image for a signal image I2, the device-noise correction image for a sample-noise correction image I4, and the dark image I5 is illustrated.

First, the image acquiring apparatus 200 divides the signal image I1, the sample-noise correction image 13, the device-noise correction image for a signal image I2, and the device-noise correction image for a sample-noise correction image I4 by the dark image I5, to perform the dark correction for removing the dark noise.

Second, the image acquiring apparatus 200 calculates the device-noise correction coefficient for each of the dark-correction device-noise correction image for a signal image I7 and the dark-corrected device-noise correction image for a sample-noise correction image I9. For example, the image acquiring apparatus 200 calculates an average brightness value of an entire image of the device-noise correction image as the device-noise correction coefficient, and generates a correction coefficient matrix that normalizes an average value obtained by dividing the brightness value of each pixel in the entire image by the average brightness value to 1. At this time, the image acquiring apparatus 200 may generate the device-noise correction coefficient based on light intensity. Moreover, the image acquiring apparatus 200 may be applied not only to the entire image but also to any field-of-view region of interest (ROI) in which a spot is detected, and may generate the device-noise correction coefficient for each of sections obtained by dividing the entire image into multiple sections. Moreover, the image acquiring apparatus 200 acquires, when the spot detection is evaluated based on a difference between the background light and a signal of a DNA spot in the line profile, a line profile of the device-noise correction image at the same coordinates as the line profile used for spot detection, calculates an average brightness value of the line profile similarly, and generates the device-noise correction coefficient that normalizes an average value obtained by dividing the brightness value of each pixel in the entire image by the average value to 1. The image acquiring apparatus 100 can identify an area of the DNA spot by image processing, such as contour extraction and feature extraction, instead of the detection method of a DNA spot using the profile described above, to perform correction.

Third, the image acquiring apparatus 200 divides the dark-correction signal image I6 by the device-noise correction coefficient for a signal image generated as described above, to generate the device-noise correction signal image I10 from which the device noise is removed. Thus, effects of unevenness of the irradiated light, scattered light from outside the device, stray light inside the device, and autofluorescence from an optical device can be removed. Similarly to calculation of the device-noise correction coefficient, the image acquiring apparatus 200 may perform the device noise correction not to the entire image, but to an arbitrary field-of-view ROI, each of regions obtained by dividing the entire image into multiple sections, and the line profile. Furthermore, similarly to the device noise correction of the dark-corrected signal image I6, the image acquiring apparatus 200 performs the device noise correction by the device-noise correction coefficient matrix of a sample-noise correction image, to generate the device-noise-corrected sample-noise correction image I11.

Fourth, the image acquiring apparatus 200 divides the device-noise-corrected signal image I10 by the device-noise-corrected sample-noise correction image I11 to generate the noise-corrected signal image I12 in which the sample noise is removed. In this case, because the device-noise-corrected signal image I10 and the device-noise-corrected sample-noise correction image I11 are not uniform in brightness value of background light to be the baseline, it is preferable that division be performed after multiplying the device-noise-corrected signal image I10 or the device-noise-corrected sample-noise correction image I11 by a constant. The constant can be calculated based on a ratio of baseline mean values of the brightness value of respective images. Moreover, a standard deviation may be calculated from the baseline of the brightness value of an image subjected to division, and the constant can be determined by a solver, for the purpose of minimizing the variable and standard deviation also.

As described above, the image acquiring apparatus 200 can generate the noise-corrected signal image I12 in which effects of dark current nonuniformity, which is fixed pattern noise in the sensor noise, unevenness of the irradiated light, scattered light from outside the device, stray light inside the device, and autofluorescence from an optical device, which are the fixed pattern noise in the device noise, nonuniform reflection caused by a surface shape of the substrate and nonuniform fluorescence in autofluorescence of the substrate, which are the fixed pattern noise in the sample noise, are removed.

### (2-3-5. White-Noise Removal Processing)

In the following, the white-noise removal processing will be explained. When light intensity of a signal and background of a sample are very low, readout noise, which is white noise not dependent on the signal intensity in the sensor noise, and shot noise due to dark current become dominant, and become larger than the fixed pattern noise described above, resulting in cancellation of the effect of fixed pattern noise removal. In general, to detect within a light intensity range that exceeds the readout noise, the signal is increased by extending exposure time, or noise reduction is performed by averaging multiple acquired images through convolution of the readout noise. However, there is a disadvantage of increasing imaging time. By using an EM-CCD that applies gain to a signal such that the light intensity of the measurement target exceeds the readout noise, or a high-sensitivity image sensor, such as a digital CMOS camera with low readout noise, as the image sensor 31, it is possible to reduce the white noise not dependent on the signal intensity in the sensor noise in imaging in short time. Moreover, these high-intensity image sensors can suppress the shot noise by cooling the sensor to low temperature.

Moreover, the shot noise due to signal current that vary depending on the signal intensity in the sensor noise occurs as noise dependent on a square root of the light intensity of the background. By extension of the exposure time, the S/N ratio is improved by noise increase in proportion to the square root for the linear increase in the light intensity of the DNA spot with respect to exposure time. Furthermore, by accumulation of multiple pieces of images, the S/N ratio is improved similarly. In the high-sensitivity image sensor described above, because the charge on the sensor becomes saturated with short exposure times, the method of accumulation mentioned above is effective.

### (2-3-6. Confirmation of Noise Reduction Effect)

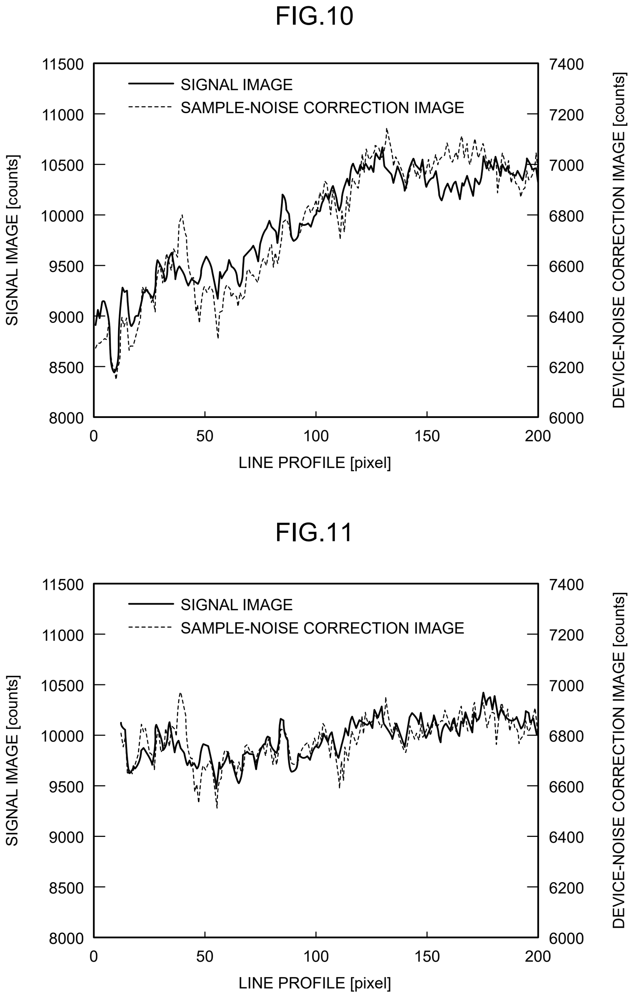

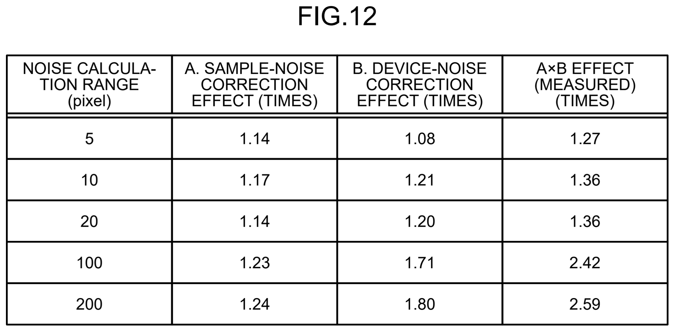

The confirmation of the noise reduction effect will be explained using FIG. 10 to FIG. 12. FIG. 10 and FIG. 11 are diagrams illustrating a specific example of a line profile on the DNA microarray 20 according to the embodiment. FIG. 12 is a diagram illustrating an example of the noise reduction effect by the sample noise correction and the device noise correction.

First, the image acquiring apparatus 200 acquires the signal image I1, the sample-noise correction image I3, the device-noise correction image for a signal image 12, the device-noise correction image for a sample-noise correction image I4, and the dark image I5. The image acquiring apparatus 200 acquires 1000 pieces of images with the exposure time of 3 seconds by the EM-CCD sensor in a state in which the laser is off, as the dark image I5, and calculates from the average image. Moreover, the image acquiring apparatus 200 excites the DNA microarray 20 by the laser having an oscillation wavelength of 532 nm, and sets the light-receiving filter with a wavelength range of 600±37.5 nm as the image signal I1. In this case, the image acquiring apparatus 200 acquires 1000 pieces of images with the exposure time of 3 seconds by the EM-CCD sensor, and calculates an average image. Furthermore, the image acquiring apparatus 200 excites the DNA microarray 20 by the laser having an oscillation wavelength of 532 nm, and sets the light-receiving filter with a wavelength range of 700±37.5 nm as the sample-noise correction image I3. In this case, the image acquiring apparatus 200 acquires 1000 pieces of images with the exposure time of 3 seconds by the EM-CCD sensor, and calculates an average image. The image acquiring apparatus 200 acquires 100 pieces each of images with the exposure time of 3 second at five different positions on the DNA microarray 20 for the unused DNA microarray 20 same as the sample with the same wavelength settings as that described above as the device-noise correction image for a signal image I2 and the device-noise correction image for a sample-noise correction image 14, and calculates an average image from the total of 500 pieces.

Next, the image acquiring apparatus 200 generates an image in which noise has been removed from each of the acquired images by the noise removal processing in FIG. 9 described above. The image acquiring apparatus 200 corrects each of the signal image I1, the sample-noise correction image I3, the device-noise correction image for a signal image I2, and the device-noise correction image for a sample-noise correction image I4 by the dark image subtraction. In this case, the image acquiring apparatus 200 sets an arbitrary line profile region at the same coordinates to the signal image I1, the sample-noise correction image I3, the device-noise correction image for a signal image I2, and the device-noise correction image for a sample-noise correction image I4.

The image acquiring apparatus 200 performs applies 19-point smoothing to the line profile respectively acquired from the device-noise correction image for a signal image I2 and the device-noise correction image for a sample-noise correction image I4. The image acquiring apparatus 200 calculates an average value of the brightness value of each of the line profile, divides the brightness value of each pixel in the entire line profile by the average brightness value, and sets the device-noise correction coefficient normalizing the average value to 1. Moreover, the image acquiring apparatus 200 generates the device-noise-corrected signal image I10 and the device-noise-corrected sample-noise correction image I11 by dividing the respective line profiles of the dark-corrected signal image I6 and the dark-corrected sample-noise correction image I8 by the device-noise correction coefficient set as described above. When the line profile before device noise correction illustrated in FIG. 10 and the line profile after device noise correction illustrated in FIG. 11 are compared, it is confirmed that the line profile after device noise correction had the baselines of the signal image I1 and the sample-noise correction image I3 are uniform, and that noise with a cycle of a several to several tens of pixels is reflected.

Furthermore, the image acquiring apparatus 200 divides, to acquire the noise-corrected signal image I12 from which the sample noise is removed, the device-noise-corrected signal image I10 by the line profile of the device-noise-corrected sample-noise correction image I11. In this case, the image acquiring apparatus 200 multiplies the sample-noise correction image I3 by the constant. The image acquiring apparatus 200 calculates a standard deviation from the baseline of the brightness value of the image after division as a constant, and determines the constant by an excel solver, for the purpose of minimizing the variable and standard deviation. A result of acquiring a noise-corrected image by the processing described above is illustrated in FIG. 12. The example in FIG. 12 is the standard deviation of the line profile calculated within a range of 5, 10, 20, 100, and 200 pixels to confirm the noise reduction effect. By performing the device noise correction and the sample noise correction (refer to "A×B EFFECT" in FIG. 12), the noise reduction effect of approximately 1.3 to 2.6 times can be confirmed. Moreover, it is also confirmed that a result of performing each of the device noise correction (refer to "B. DEVICE NOISE CORRECTION" in FIG. 12) alone also shows a large noise reduction effect, but it is confirmed that a larger noise reduction effect is obtained by performing both of the corrections. Particularly, it is confirmed that the effect of device noise correction becomes large in areas with a large number of pixels in which an influence of a slope of the brightness baseline is significant.

### (2-3-7. Modification of Image-Data Acquisition Processing and Noise Reduction Processing)

In the following, as a modification of the noise removal processing, a modification in which the dark image and the device-noise correction image is not necessary, a modification in which the dark correction is not necessary, and a modification in which the device noise correction is not necessary will be explained sequentially.

### (2-3-7-1. Modification in which Dark Image and Device-Noise Correction Imaged is Unnecessary)

When it is assumed that patterns of the dark noise and the device noise do not vary for each measurement of a sample, it is not necessary to acquire the dark image I5 and the device-noise correction images I2, I4 for each measurement of the sample. An image having a pattern of noise to be removed can be prepared in advance. Furthermore, in the dark image subtraction, the white noise included in the signal image I1 and the like and the white noise included in the dark image I5 increase by a factor of √2 due to error propagation. To reduce the white noise included in the dark image I5, it is preferable to use an image obtained by averaging the multiple dark images I5. Because the device-noise correction images I2, I4 also include the white noise in the device-noise correction coefficient, it is preferable to use an image obtained by averaging the multiple device-noise correction images I2, I4.

### (2-3-7-2. Modification in Which Dark Correction is Unnecessary)

When a magnitude of baseline brightness is similar between the signal image I1 and the sample-noise correction image I3, it is not necessary to perform the dark noise subtraction. The dark-noise is included in both the signal image I1 and the sample-noise correction image I3. Therefore, in division of the image in the sample noise correction, the dark noise is divided to be corrected. Therefore, acquisition of the dark image I5 and the dark correction in the calculation processing become unnecessary.

### (2-3-7-3. Modification in Which Device Noise Correction is Unnecessary)

When the device configurations at the time of acquiring the signal image I1 and the sample-noise correction image I3 are similar, and the pattern of unevenness of irradiated light is the same, it is not necessary to perform the device noise correction. Because it is possible to correct a slope of the baseline of the image brightness in the subtraction processing of the signal image I1 and the sample-noise correction image I3 in the sample noise correction, the device noise correction in the acquisition of the device-noise correction images 12, I4 and calculation processing becomes unnecessary. In this case, unevenness in light intensity of the DNA spot caused by the unevenness in light intensity of illumination light due to nonuniformity of the illumination light is not corrected.

### [3. Flow of Processing of Image Acquiring System 2000]

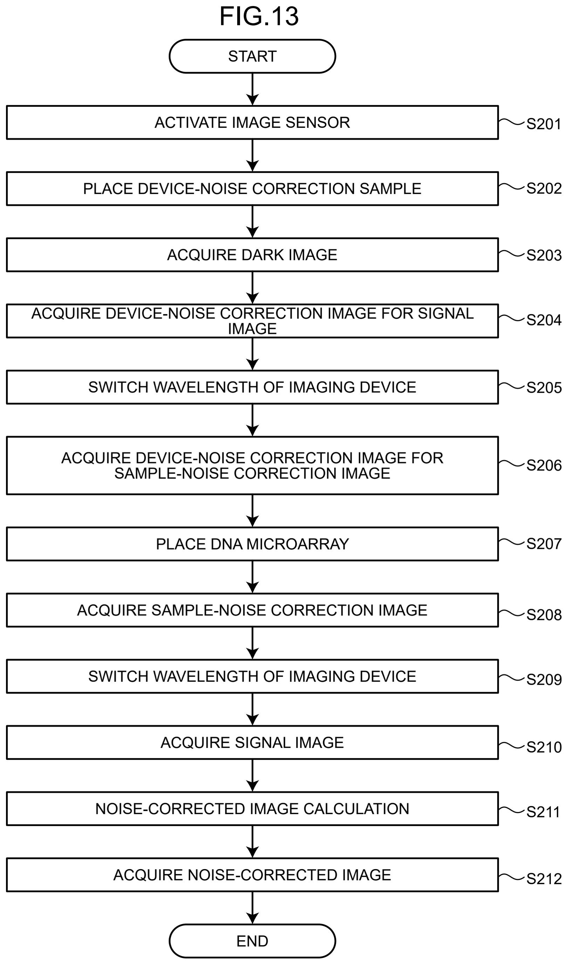

A flow of processing of the image acquiring system 2000 according to the embodiment will be explained using FIG. 13. FIG. 13 is a flowchart illustrating an example of the flow of the image acquisition processing according to the embodiment. In the following, the acquisition processing of the dark image I5 at steps S201 to S203, the acquisition processing of the device-noise correction image for a signal image I2 at step S204, the acquisition processing of the device-noise correction image for a sample-noise correction image I4 at steps S205 to S206, the acquisition processing of the sample-noise correction image I3 at steps S207 to S208, the acquisition processing of the signal image I1 at steps S209 to S210, and the acquisition processing of the noise signal image I12 at steps S211 to S212 will be explained sequentially. The processing at steps S201 to S212 described below can be performed in different order. Moreover, out of the processing at steps S201 to S212 described below, some processing may be omitted.

### (3-1. Acquisition Processing of Dark Image I5)

First, the imaging device 2 activates the image sensor 31 (step S201). Second, the imaging device 2 places a sample for the device-noise correction on the stage 32 (step S202). At this time, the imaging device 2 sets the third imaging condition in which the first laser-light source 21, which is the laser light source for a signal image, and the second laser-light source 22, which is the laser light source for a sample-noise correction image, are both turned off. Third, the image acquiring apparatus 200 acquires the dark image I5 captured by the imaging device 2 (step S203).

### (3-2. Acquisition Processing of Device-Noise Correction Image for Signal Image)

First, the imaging device 2 sets the first imaging condition in which the first laser-light source 21 is on and the second laser-light source 22 is off, and selects the excitation wavelength and the light receiving wavelength by arranging the first dichroic mirror 26, which is the dichroic mirror for a signal image, and the first light-receiving filter 28, which is the light receiving filter for a signal image. Second, the image acquiring apparatus 200 acquires the device-noise correction image for a signal image I2 captured by the imaging device 2 (step S204).

### (3-3. Acquisition Processing of Device-Noise Correction Image for Sample-Noise Correction Image I4)

First, the imaging device 2 sets the second imaging condition in which the first laser-light source 21 is off and the second laser-light source 22 is on, and switches the excitation wavelength and the light receiving wavelength by arranging the second dichroic mirror 27, which is the dichroic mirror for a sample-noise correction image, and the second light-receiving filter 29, which is the light receiving filter for a sample-noise correction image (step S205). Second, the image acquiring apparatus 200 acquires the device-noise correction image for a sample-noise correction image I4 (step S206).

### (3-4. Acquisition Processing of Sample-Noise Correction Image 13)

First, the imaging device 2 places the DNA microarray 20, which is a measurement target sample, replacing the device-noise correction sample on the stage 32 (step S207). Second, the image acquiring apparatus 200 acquires the sample-noise correction image I3 captured by the imaging device 2 under the imaging condition with the device configuration at step S206 (step S208).

### (3-5. Acquisition Processing of Signal Image I1)

First, the imaging device 2 switches the excitation wavelength and the light receiving wavelength by changing the imaging condition and the device configuration again to the state at step S204 (step S209). Second, the image acquiring apparatus 200 acquires the signal image I1 captured by the imaging device 2 (step S210).

### (3-6. Acquisition Processing of Noise Signal Image I12)

First, the image acquiring apparatus 200 performs the calculation processing of noise correction from the signal image I1, the sample-noise correction image I3, the device-noise correction image for a signal image I2, the device-noise correction image for a sample-noise correction image I4, and the dark image I5 (step S211). Second, the image acquiring apparatus 200 acquires the noise-corrected image I12 in which noise is removed (step S212).

### (4. Effect of Embodiment)

Finally, effects of the embodiment will be explained. In the following, effects 1 to 8 corresponding to the processing according to the embodiment will be explained.

### (4-1. Effect 1)

First, in the processing according to the embodiment described above, the image acquiring apparatus 200 acquires the signal image I1 obtained by capturing the DNA microarray 20 by the imaging device 2 under the first imaging condition in which a predetermined excitation wavelength and a predetermined light receiving wavelength are used, acquires the sample-noise correction image I3 obtained by capturing the DNA microarray 20 by the imaging device 2 under the second imaging condition that differs in at least the excitation wavelength and the light receiving wavelength from the first imaging condition, and generates the noise-corrected signal image I12 in which the sample noise included in the signal image I1 is removed using the sample-noise correction image I3. Therefore, the processing according to the embodiment enables to acquire a fluorescence image with high detection accuracy by removing sample noise in the signal image I1 in the DNA microarray method.

### (4-2. Effect 2)

Second, in the processing according to the embodiment described above, the image acquiring apparatus 200 further acquires the device-noise correction image for a signal image I2 obtained by capturing a device-noise correction sample different from the DNA microarray 20 by the imaging device 2 under the first imaging condition, and generates the noise-corrected signal image I12 in which device noise included in the signal image I1 is further removed, by using the device-noise correction image for a signal image I2. Therefore, the processing according to the embodiment enables to acquire a fluorescence image with high detection accuracy by further removing device noise in the signal image I1 in the DNA microarray method.

### (4-3. Effect 3)

Third, in the processing according to the embodiment described above, the image acquiring apparatus 200 further acquires the device-noise correction image for a sample-noise correction image I4 in which a device-noise correction sample is captured by the imaging device 2 under the second imaging condition, further removes device noise included in the sample-noise correction image I3 by using the device-noise correction image for a sample-noise correction image I4, and generates the noise-corrected signal image I12 in which the device noise included in the signal image I1 is removed using the sample-noise correction image I3 from which the device noise has been removed. Therefore, the processing according to the embodiment enables to acquire a fluorescence image with high detection accuracy by further removing device noise in the sample-noise correction image I3 in the DNA microarray method.

### (4-4. Effect 4)

Fourth, in the processing according to the embodiment described above, the image acquiring apparatus 200 further acquires the dark image I5 captured by the imaging device 2 under the third imaging condition in which excitation light is not irradiated, further removes dark noise included in the signal image I1, the sample-noise correction image I3, the device-noise correction image for a signal image I2, and the device-noise correction image for a sample-noise correction image I4, and generates the noise-corrected signal image I12. Therefore, the processing according to the embodiment enables to acquire a fluorescence image with high detection accuracy by further removing dark noise in the signal image I1 in the DNA microarray method.

### (4-5. Effect 5)

Fifth, in the processing according to the embodiment described above, the image acquiring apparatus 200 further acquires the signal image I1 in which the DNA microarray 20 is captured by the imaging device 2 under the first imaging condition in which the first laser-light source 21 and the first light-receiving filter 28 are used, acquires the sample-noise correction image I3 in which the DNA microarray 20 is captured by the imaging device 2 under the second imaging condition in which at least one of the second laser-light source 22 and the second light-receiving filter 29 is used, acquires the device-noise correction image for a signal image I2 in which a sample not containing fluorescence emitted by the DNA microarray 20 or a mirror is captured as the device-noise correction sample by the imaging device 2 under the first imaging condition, captures the device-noise correction image for a sample-noise correction image I4 in which a device-noise correction sample is captured by the imaging device 2 under the second imaging condition, and acquires the dark image I5 in which the DNA microarray 20 or the device-noise correction sample is captured by the imaging device 2 under the third imaging condition in which excitation light is not irradiated from neither the first laser-light source 21 nor the second laser-light source 22. Therefore, the processing according to the embodiment enables to acquire a fluorescence image with high detection accuracy by acquiring various kinds of images to remove the sample noise the device noise, and the dark noise in the DNA microarray method.

### (4-6. Effect 6)

Sixth, in the processing according to the embodiment described above, the image acquiring apparatus 200 further calculates a device-noise correction coefficient for a signal image based on a brightness value or light intensity from the dark-corrected device-noise correction image for a signal image I7, generates the device-noise corrected fluorescence image I10 obtained by removing the device noise from the signal image I1 by dividing the dark-correction signal image I1 by the device-noise correction coefficient for a signal image, calculates the device-noise correction coefficient for a sample-noise correction image based on a brightness value or light intensity from the dark-corrected device-noise correction image for a sample-noise correction image I9, generates the device-noise-corrected sample-noise correction image I11 obtained by removing the device noise from the sample-noise correction image I3 by dividing the dark-corrected sample-noise correction image I8 by the device-noise correction coefficient for a sample-noise correction image, and generates the noise-corrected signal image I12 obtained by removing the sample noise from the device-noise-corrected signal image I10 by dividing the device-noise-corrected signal image I10 by the device-noise-corrected sample-noise correction image I11. Therefore, the processing according to the embodiment enables to acquire a fluorescence image with high detection accuracy by performing various calculations to remove the sample noise, the device noise, and the dark noise in the DNA microarray method.

### (4-7. Effect 7)

Seventh, in the processing according to the embodiment described above, the image acquiring apparatus 200 acquires the signal image I1 captured by using the imaging device 2 equipped with the image sensor 31 that applies gain to a signal so that intensity of light emitted by the DNA microarray 20 is equal to or higher than readout noise, or the image sensor 31 in which readout noise is equal to or lower than a predetermined value. Therefore, the processing according to the embodiment enables to acquire a fluorescence image with high detection accuracy by acquiring an image from which white noise is removed in the DNA microarray method.

### [Application Example of Embodiment]

In the following, an application example of the embodiment will be explained. The present invention is not limited to an application example of the embodiment explained below.

The embodiment can be applied to biomacromolecules such as nucleic acid, glycan, and protein as a specific target molecule.

The embodiment can be applied to image acquisition and analysis of a DNA microarray using a fluorescence method, a chemiluminescence method, and a colorimetric method.

The embodiment can be applied to a nucleic acid sequence measurement device in which a probe solid-phase-immobilized on a substrate is labeled with a signal-generating source such as a fluorescent molecule, detection of an unlabeled target using a signaling array probe, and microarray detection of a nucleic acid sequence measurement device that does not need washing, in a DNA microarray and the like.

The embodiment can be applied to detection of a nucleic acid sequence measurement device that captures and observes a target molecule that are labeled with a signal-generating source, such as a fluorescent molecule, using a probe solid-phase-immobilized on a substrate, in a DNA microarray and the like.

The embodiment can be applied to the detection of a nucleic acid sequence measurement device that captures and observes a target molecule bound to a labeling molecule labeling a signal-generating source, such as a fluorescent molecule that specifically binds to the target molecule, with a probe solid-phase-immobilized on a substrate in a DNA microarray and the like.

The embodiment can be applied to gene expression analysis, genotyping, and base sequence analysis when targeting a nucleic acid.

The embodiment can be applied to glycan profiling when targeting a glycan.

The embodiment can be applied to antibody profiling and protein-protein interaction analysis when targeting a protein.

The embodiment can be applied to drug discovery and development, biomarker detection, disease diagnosis, disease prevention and prognosis management, biomarker discovery, diagnostic method development, antibody-antigen exploration, plant variety identification, genetic testing of livestock and agricultural products, genetically modified organism testing in food, allergen labeling testing, microbiological detection and microbiological identification in environmental hygiene testing, microbiological testing in product quality control, fermentation process monitoring, fermentation starter culture management, useful microorganism discovery in microbial materials, and hazard microorganism characterization in pharmaceutical and food manufacturing processes, as various industrial applications.

### [System]

The processing procedures, control procedures, specific names, and various kinds of data and parameters described in the above document and the drawings can be arbitrarily changed unless otherwise specified.

Furthermore, the respective components of the respective devices illustrated in the drawings are of functional concept and are not necessarily required to be configured physically as illustrated. That is, specific forms of distribution and integration of the respective devices are not limited to the ones illustrated. That is, all or some thereof can be configured to be distributed or integrated functionally or physically in arbitrary units according to various kinds of loads, usage conditions, and the like.

Furthermore, as for the respective processing functions performed by the respective devices, all or an arbitrary part thereof can be implemented by a CPU and a computer program that is analyzed and executed by the CPU, or can be implemented as hardware by wired logic.

### [Hardware]

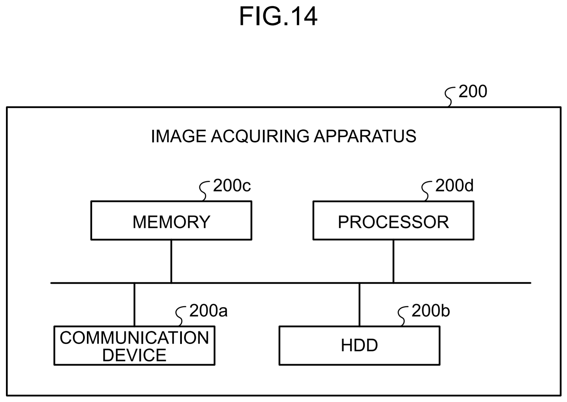

Next, a hardware configuration of the image acquiring apparatus 200 will be explained. FIG. 14 is a diagram illustrating a hardware configuration example according to the embodiment. As illustrated in FIG. 14, the image acquiring apparatus 200 includes a communication device 200a, an HDD (hard disk drive) 200b, a memory 200c, and a processor 200d. Moreover, respective components illustrated in FIG. 14 are connected to one another through a bus and the like.

The communication device 200a is a network interface card or the like, and performs communication with other servers. The HDD 200b stores a program or database (DB) that operates functions illustrated in FIG. 7.

The processor 200d retrieves a program to perform processing similarly to each of the processing units illustrated in FIG. 7 from the HDD 200b or the like, and loads it to the memory 200c, thereby operating processes to implement the respective functions explained in FIG. 7 and the like. For example, this process implements functions similar to those of the respective processing units included in the image acquiring apparatus 200. Specifically, the processor 200d reads a program having a function equivalent to those of the acquiring unit 231, the generating unit 232, and the like from the HDD 200b or the like. The processor 200d performs a process to implement processing similar to that of the acquiring unit 231, the generating unit 232, and the like.

As described, the image acquiring apparatus 200 operates as a device that performs various kinds of processing methods by reading and executing a program. Moreover, the image acquiring apparatus 200 can implement a function similar to that of the embodiment described above by reading the program described above from a recording medium by a medium reader device, and by executing the read program described above also. The program in other embodiments is not limited to be executed by the image acquiring apparatus 200. For example, also when other computers or servers execute the program, or when these collaborate to execute the program, the present invention can be similarly applied.

This program can be distributed through a network such as the Internet. Furthermore, this program can be recorded on a computer-readable recording medium, such as a hard disk, a flexible disk (FD), a CD-ROM, a MO (magneto optical disk), and a DVD (digital versatile disk), and can be executed by being read by a computer from the recording medium.

### [Others]

Some examples of combinations of the disclosed technical features are described below.
(1) An image acquiring apparatus that includes an acquiring unit that acquires a fluorescence image in which a measurement target sample is captured by an imaging device under a first imaging condition using a predetermined excitation wavelength and a predetermined light receiving wavelength, and that acquires a first correction image in which the measurement target sample is captured by the imaging device under a second imaging condition in which at least one of the excitation wavelength and the light receiving wavelength is different from those of the first imaging condition; and a generating unit that generates a noise-corrected fluorescence image in which sample noise originating from the measurement target sample included in the fluorescence image is removed, by using the first correction image.
(2) The image acquiring apparatus according to (1), wherein the acquiring unit further acquires a second correction image in which a correction sample that is different from the measurement target sample is captured by the imaging device under the first imaging condition, and the generating unit generates the noise-corrected fluorescence image in which device noise originating from the imaging device included in the fluorescence image is further removed by using the second correction image.
(3) The image acquiring apparatus according to (2), wherein the acquiring unit further acquires a third correction image in which the correction sample is captured by the imaging device under the second imaging condition, and the generating unit further removes the device noise included in the first correction image by using the third correction image, and generates the noise-corrected fluorescence image from which the device noise included in the fluorescence image is further removed by using the first correction image from which the device noise has been removed.
(4) The image acquiring apparatus according to (3), wherein the acquiring unit further acquires a fourth correction image captured by the imaging device under a third imaging condition in which excitation light is not irradiated, and the generating unit further removes dark noise originating from dark current of the imaging device included in the fluorescence image, the first correction image, the second correction image, and the third correction image, and generates the noise-corrected fluorescence image.
(5) The image acquiring apparatus according to (4), wherein the acquiring unit acquires the fluorescence image in which the measurement target sample is captured by the imaging device under the first imaging condition using the first light source that irradiates excitation light of a predetermined wavelength and the first filter to select fluorescence of a predetermined wavelength, acquires the first correction image in which the measurement target sample is captured by the imaging device under the second imaging condition using at least one of the second light source that irradiates excitation light having a wavelength different from that of the first light source and the second filter to select a fluorescence of a wavelength different from that of the first filter, acquires the second correction image in which any one of a sample not containing fluorescence emitted by the measurement target sample and a mirror is captured as the correction sample by the imaging device under the first imaging condition, acquires the third correction image in which the correction sample is captured by the imaging device under the second imaging condition, and acquires the fourth correction image in which the measurement target sample or the correction sample is captured by the imaging device under the third imaging condition in which excitation light is not irradiated neither from the first light source nor the second light source.
(6) The image acquiring apparatus according to (4) or (5), wherein the generating unit calculates a first device-noise correction coefficient based on a brightness value or light intensity from the second correction image from which the dark noise has been removed, and generates a device-noise-corrected fluorescence image in which the device noise is removed from the fluorescence image by dividing the florescence image from which the dark noise has been removed by the first device-noise correction coefficient, calculates a second device-noise correction coefficient based on a brightness value or light intensity from the third correction image from which the dark noise has been removed, and generates a fifth correction image in which the device noise is removed from the first correction image by dividing the first correction image from which the dark noise has been removed by the second device-noise correction coefficient, and generates the noise-correction fluorescence image in which the sample noise is removed from the device-noise correction fluorescence image by dividing the device-noise correction fluorescence image by the fifth correction image.
(7) The image acquiring apparatus according to any one of (1) to (6), wherein the acquiring unit acquires the fluorescence image captured by using the imaging device having an image sensor that applies gain to a signal such that intensity of light emitted by the measurement target sample becomes equal to or larger than readout noise, or an image sensor in which readout noise is equal to or lower than a predetermined value.
(8) The image acquiring apparatus according to any one of (1) to (7), wherein the measurement target sample is a DNA microarray.
(9) An image acquiring method of a computer performing processing of acquiring a fluorescence image in which a measurement target sample is captured by an imaging device under a first imaging condition using a predetermined excitation wavelength and a predetermined light receiving wavelength, acquiring a first correction image in which the measurement target sample is captured by the imaging device under a second imaging condition in which at least one of the excitation wavelength and the light receiving wavelength is different from those of the first imaging condition; and generating a noise-corrected fluorescence image in which sample noise originating from the measurement target sample included in the fluorescence image is removed, by using the first correction image.
(10) An image acquiring program that causes a computer to execute acquiring a fluorescence image in which a measurement target sample is captured by an imaging device under a first imaging condition using a predetermined excitation wavelength and a predetermined light receiving wavelength, acquiring a first correction image in which the measurement target sample is captured by the imaging device under a second imaging condition in which at least one of the excitation wavelength and the light receiving wavelength is different from those of the first imaging condition; and generating a noise-corrected fluorescence image in which sample noise originating from the measurement target sample included in the fluorescence image is removed, by using the first correction image.

### Reference Signs List

- 1: IMAGING DEVICE

- 10: LASER LIGHT SOURCE
- 11: MIRROR
- 12: DICHROIC MIRROR
- 13: LIGHT RECEIVING FILTER
- 14: LIGHT-RECEIVING OPTICAL SYSTEM
- 15: IMAGE SENSOR
- 16: STAGE
- 20: DNA MICROARRAY
- 100: IMAGE ACQUIRING APPARATUS
- 1000: IMAGE ACQUIRING SYSTEM
- 2: IMAGING DEVICE
- 21: FIRST LASER-LIGHT SOURCE
- 22: SECOND LASER-LIGHT SOURCE
- 23: FIRST MIRROR
- 24: SECOND MIRROR
- 25: THIRD MIRROR
- 26: FIRST DICHROIC MIRROR
- 27: SECOND DICHROIC MIRROR
- 28: FIRST LIGHT-RECEIVING FILTER
- 29: SECOND LIGHT-RECEIVING FILTER
- 30: LIGHT-RECEIVING OPTICAL SYSTEM
- 31: IMAGE SENSOR
- 32: STAGE
- 200: IMAGE ACQUIRING APPARATUS
- 210: COMMUNICATION UNIT
- 220: STORAGE UNIT
- 230: CONTROL UNIT
- 231: ACQUIRING UNIT
- 232: GENERATING UNIT
- 2000: IMAGE ACQUIRING SYSTEM

## Claims

1. An image acquiring apparatus comprising:
an acquiring unit that acquires a fluorescence image in which a measurement target sample is captured by an imaging device under a first imaging condition using a predetermined excitation wavelength and a predetermined light receiving wavelength, and
that acquires a first correction image in which the measurement target sample is captured by the imaging device under a second imaging condition in which at least one of the excitation wavelength and the light receiving wavelength is different from those of the first imaging condition; and
a generating unit that generates a noise-corrected fluorescence image in which sample noise originating from the measurement target sample included in the fluorescence image is removed, by using the first correction image.

2. The image acquiring apparatus according to claim 1, wherein
the acquiring unit further acquires a second correction image in which a correction sample that is different from the measurement target sample is captured by the imaging device under the first imaging condition, and
the generating unit generates the noise-corrected fluorescence image in which device noise originating from the imaging device included in the fluorescence image is further removed by using the second correction image.

3. The image acquiring apparatus according to claim 2, wherein
the acquiring unit further acquires a third correction image in which the correction sample is captured by the imaging device under the second imaging condition, and
the generating unit further removes the device noise included in the first correction image by using the third correction image, and generates the noise-corrected fluorescence image from which the device noise included in the fluorescence image is further removed by using the first correction image from which the device noise has been removed.

4. The image acquiring apparatus according to claim 3, wherein
the acquiring unit further acquires a fourth correction image captured by the imaging device under a third imaging condition in which excitation light is not irradiated, and
the generating unit further removes dark noise originating from dark current of the imaging device included in the fluorescence image, the first correction image, the second correction image, and the third correction image, and generates the noise-corrected fluorescence image.

5. The image acquiring apparatus according to claim 4, wherein
the acquiring unit acquires the fluorescence image in which the measurement target sample is captured by the imaging device under the first imaging condition using the first light source that irradiates excitation light of a predetermined wavelength and the first filter to select fluorescence of a predetermined wavelength,
acquires the first correction image in which the measurement target sample is captured by the imaging device under the second imaging condition using at least one of the second light source that irradiates excitation light having a wavelength different from that of the first light source and the second filter to select a fluorescence of a wavelength different from that of the first filter,
acquires the second correction image in which any one of a sample not containing fluorescence emitted by the measurement target sample and a mirror is captured as the correction sample by the imaging device under the first imaging condition,
acquires the third correction image in which the correction sample is captured by the imaging device under the second imaging condition, and
acquires the fourth correction image in which any one of the measurement target sample and the correction sample is captured by the imaging device under the third imaging condition in which excitation light is not irradiated neither from the first light source nor the second light source.

6. The image acquiring apparatus according to claim 5, wherein
the generating unit calculates a first device-noise correction coefficient based on any one of a brightness value and light intensity from the second correction image from which the dark noise has been removed, and generates a device-noise-corrected fluorescence image in which the device noise is removed from the fluorescence image by dividing the florescence image from which the dark noise has been removed by the first device-noise correction coefficient,
calculates a second device-noise correction coefficient based on any one of a brightness value and light intensity from the third correction image from which the dark noise has been removed, and generates a fifth correction image in which the device noise is removed from the first correction image by dividing the first correction image from which the dark noise has been removed by the second device-noise correction coefficient, and
generates the noise-correction fluorescence image in which the sample noise is removed from the device-noise correction fluorescence image by dividing the device-noise correction fluorescence image by the fifth correction image.

7. The image acquiring apparatus according to claim 6, wherein
the acquiring unit acquires the fluorescence image captured by using the imaging device having any one of an image sensor that applies gain to a signal such that intensity of light emitted by the measurement target sample becomes equal to or larger than readout noise, and an image sensor in which readout noise is equal to or lower than a predetermined value.

8. The image acquiring apparatus according to any one of claims 1 to 7, wherein
the measurement target sample is a DNA microarray.

9. An image acquiring method of a computer performing processing of:
acquiring a fluorescence image in which a measurement target sample is captured by an imaging device under a first imaging condition using a predetermined excitation wavelength and a predetermined light receiving wavelength,
acquiring a first correction image in which the measurement target sample is captured by the imaging device under a second imaging condition in which at least one of the excitation wavelength and the light receiving wavelength is different from those of the first imaging condition; and
generating a noise-corrected fluorescence image in which sample noise originating from the measurement target sample included in the fluorescence image is removed, by using the first correction image.

10. An image acquiring program that causes a computer to execute:
acquiring a fluorescence image in which a measurement target sample is captured by an imaging device under a first imaging condition using a predetermined excitation wavelength and a predetermined light receiving wavelength,
acquiring a first correction image in which the measurement target sample is captured by the imaging device under a second imaging condition in which at least one of the excitation wavelength and the light receiving wavelength is different from those of the first imaging condition; and
generating a noise-corrected fluorescence image in which sample noise originating from the measurement target sample included in the fluorescence image is removed, by using the first correction image.
